(19) Europäisches Patentamt
European Patent Office
Office européen des brevets

(11) EP 1 871 522 B1

(12) **EUROPEAN PATENT SPECIFICATION**

(45) Date of publication and mention of the grant of the patent:
**15.10.2008 Bulletin 2008/42**

(21) Application number: **06761901.5**

(22) Date of filing: **24.03.2006**

(51) Int Cl.:
*B01J 23/00* (2006.01)     *B01J 23/28* (2006.01)
*B01J 23/20* (2006.01)     *B01J 23/18* (2006.01)
*B01J 37/02* (2006.01)     *B01J 37/08* (2006.01)
*C07C 51/215* (2006.01)     *C07C 57/04* (2006.01)

(86) International application number:
**PCT/EP2006/003685**

(87) International publication number:
**WO 2006/100128 (28.09.2006 Gazette 2006/39)**

(54) **PROCESS FOR PREPARING IMPROVED CATALYSTS FOR SELECTIVE OXIDATION OF PROPANE INTO ACRYLIC ACID**

VERFAHREN ZUR HERSTELLUNG VERBESSERTER KATALYSATOREN ZUR SELEKTIVEN OXIDIERUNG VON PROPAN ZU ACRYLSÄURE

PROCEDE DE PREPARATION DE CATALYSEURS AMELIORES POUR L'OXYDATION SELECTIVE DE PROPANE EN ACIDE ACRYLIQUE

(84) Designated Contracting States:
**AT BE BG CH CY CZ DE DK EE ES FI FR GB GR HU IE IS IT LI LT LU LV MC NL PL PT RO SE SI SK TR**

(30) Priority: **25.03.2005 EP 05290665**

(43) Date of publication of application:
**02.01.2008 Bulletin 2008/01**

(73) Proprietor: **Arkema France**
**92800 Puteaux (FR)**

(72) Inventors:
• **Dubois, Jean-Luc**
  **F-69390 Millery (FR)**
• **Ueda, Wataru**
  **Machida-shi**
  **Tokyo 194-0044 (JP)**
• **Endo, Yusuke**
  **Ichihara-shi**
  **CHIBA 299-0124 (JP)**

(74) Representative: **Bonnel, Claudine**
**ARKEMA FRANCE**
**Département Propriété Industrielle**
**420, rue d'Estienne d'Orves**
**92705 Colombes Cedex (FR)**

(56) References cited:
**EP-A- 0 608 838          EP-A- 0 997 454**
**EP-A- 1 193 240**

• **PATENT ABSTRACTS OF JAPAN vol. 1998, no. 06, 30 April 1998 (1998-04-30) & JP 10 028862 A (MITSUBISHI CHEM CORP), 3 February 1998 (1998-02-03) -& JP 10 028862 A (MITSUBISHI CHEM CORP) 3 February 1998 (1998-02-03)**
• **VITRY D ET AL: "Mo-V-Te-(Nb)-O mixed metal oxides prepared by hydrothermal synthesis for catalytic selective oxidations of propane and propene to acrylic acid" APPLIED CATALYSIS A: GENERAL, ELSEVIER SCIENCE, AMSTERDAM, NL, vol. 251, no. 2, 30 September 2003 (2003-09-30), pages 411-424, XP004458147 ISSN: 0926-860X**

**Description**

The invention concerns the selective oxidation of propane into acrylic acid using highly selective catalysts and relates more particularly to a process for preparing these improved catalysts and their use for the production of acrylic acid from propane.

The selective oxidation of light alkanes into oxygenated products is a very attractive way for the chemical utilization of large natural gas resources. Nowadays, there is an increasing interest in the development of a process for direct oxidation of propane to acrylic acid as an alternative to the two-step conventional industrial process. In this selective oxidation field, catalysts performances relating to the conversion of propane and selectivity to acrylic acid are the most often too limited; and there is a need to improve catalytic performances of solids generally used for this reaction.

The patent application EP-A-608838 describes catalysts containing a mixed metal oxide comprising as essential components, Mo, V, Te, O and X, wherein X is at least one element selected from the group consisting of niobium, tantalum, tungsten, titanium, aluminium, zirconium, chromium, manganese, iron, ruthenium, cobalt, rhodium, nickel, palladium, platinum, antimony, bismuth, boron, indium and cerium, these elements being present in determined proportions. When a MoVTeNbO mixed metal oxide is to be prepared, an aqueous solution of telluric acid, an aqueous solution of ammonium niobium oxalate and a solution or slurry of ammonium paramolybdate are sequentially added to an aqueous solution containing ammonium metavanadate, the mixture in then dried and finally the remaining dried product is calcined. Preferred is the catalyst which exhibits the main peaks at $2\theta = 22.1°$ and $28.2°$ in the X-ray diffraction pattern. This catalyst is claimed to achieve acrylic acid yields substantially better than the conventional methods. With a comparative MoVTeO mixed metal oxide, no formation of acrylic acid was detected.

MoVMO catalysts (M=Al, Ga, Bi, Sb and Te), prepared by hydrothermal synthesis, have also been studied in the partial oxidation of propane (Applied Catalysis A:200 (2000) 135-143). However, MoVSbO catalysts present selectivity to acid acrylic lower than that obtained on Te-based catalyst, although their catalyst performance has partially been enhanced by grinding the catalyst after calcination step.

Japanese Laid-Open Patent application Publication N°10-330343 discloses catalysts useful for production of nitriles by vapour phase oxidation of an alkane. These catalysts having a crystalline structure are represented by the formula $MO_aV_bSb_cX_xO_n$ wherein X is one or more kinds of metallic elements selected from Ti, Zr, Nb, Ta, Cr....A precursor is first prepared by addition of solutions or suspensions containing respectively a source of antimony and a source of vanadium, then addition of a solution or suspension containing a specific amount of molybdenum and addition of the element X as powder form or solution. This precursor is then dried and calcined. The solid obtained is a mixed metal oxide having specific main and powdery X-ray diffraction peaks corresponding to a mixture of an orthorhombic phase and hexagonal phase material. Further treatments as the washing with a solvent selected from aqueous oxalic acid, ethylene glycol or aqueous hydrogen peroxide, allow separating the orthorhombic phase material as an improved catalyst.

It is known by US patent 6,060,422 to use a metal oxide containing metallic elements Mo, V, Sb and A, wherein A can be Nb or Ta for use in producing acrylic acid by the gas-phase catalytic oxidation of propane. The process for producing this catalyst comprises a step (1) of reacting $V^{+5}$ with $Sb^{+3}$ in an aqueous medium at a temperature not lower than 70°C in the presence of $Mo^{+6}$ and, during or after the reaction, bubbling either molecular oxygen or a gas containing molecular oxygen into the reaction mixture, and a step (2) of adding a compound containing the element A to the reaction product obtained in step (1), mixing the ingredients to obtain a homogeneous mixture, and calcining the resultant mixture. In this process the metal A is added to either the dispersion which is the reaction mixture resulting from the above reaction and contains Mo, V and Sb, or a solid matter obtained by subjecting the dispersion to evaporation to dryness. The metal oxide obtained by this process reveals peak at a diffraction angle $2\theta$ of 28.1. In the catalytic production of acrylic acid from propane, selectivity for acrylic acid of 29.5% is obtained at a reaction temperature of 400°C. Higher selectivity can be performed while using as catalyst a metal oxide obtained by depositing at least one compound which contains an element B selected from the group consisting of Na, K, Rb, Cs, P and As on the oxide obtained above.

In US patent application 2003/0013904, there are provided catalysts wherein the performance for the vapour phase oxidation of an alkane to an unsaturated carboxylic acid is enhanced by doping catalysts comprising a mixed metal oxide which can be a MoV(Te or Sb)(Nb or Ta) mixed metal oxide, with a metal or a combination of metals. The preferred dopants are Pd or Pd-Au alloys. In a first step, a catalyst precursor admixture is formed by admixing metal compounds and at least one solvent to form the admixture that may be a slurry, solution or combination thereof. Liquids are then removed and the resulting precursor admixture is calcined. The dopants are introduced prior to, during or after calcination by sputtering.

In US patent application 2002/0183198, a mixed metal oxide, which may be an orthorhombic phase material, is improved as a catalyst for the production of unsaturated carboxylic acids from alkanes, by a process comprising contacting with a liquid contact member selected from the group consisting of organic acids, alcohols, inorganic acids and hydrogen peroxide.

Japanese Patent application JP 10-28862 discloses a process to obtain an improved metal oxide catalyst that is effective in gas-phase catalytic-oxidation reactions for hydrocarbons by which acrylonitrile or acrylic acid can be prepared. This catalyst is obtained by impregnating a compound metal oxide represented by the following formula :$Mo_a V_b X_x Z_z O_n$, in which, X is Te and/or Sb, Z is at least one element chosen from Nb, Ta, W, Ti, Al, Zr, Cr, Mn, Fe, Ru, Co, Rh, Ni, Pd, Pt, Bi, B, In and Ce, with a solution containing at least one element chosen from the group of tungsten, molybdenum, chromium, zirconium, titanium, niobium, tantalum, vanadium, boron, bismuth, tellurium, palladium, cobalt, nickel, iron, phosphor, silicon, rare earth elements, alkali metals and alkaline earth metals. The compound metal oxide is first prepared according to dry-up method, by preparing an aqueous solution or slurry containing all components followed by drying and calcinations, this method being desirable to obtain a catalyst excellent in activity.

The prior art continues to seek ways to improve the performances of mixed metal oxide catalysts for the production of acrylic acid from propane.

It has now been found surprisingly that the performance of a Mo-V-(Te and/or Sb)-(Nb and/or Ta)-O mixed metal oxide catalyst having the formula (I) :

$$Mo_1V_a(Te \text{ and/or } Sb)_b(Nb \text{ and/or } Ta)_cSi_dO_x \qquad (I)$$

Wherein a is between 0.006 and 1
b is between 0.006 and 1
c is between 0.001 and 0.5
d is between 0 and 3.5

and x is dependent on the oxidation state of the other elements,
may be improved for the oxidation of propane to acrylic acid by carrying out a new process for its preparation. This process comprises addition of a doping agent, constituted by at least one component selected from niobium and tantalum to a mainly orthorhombic phase prepared by hydrothermal synthesis method of a Mo-V-Sb (and/or Te)-O catalyst, optionally containing Nb and/or Ta and/or Si The addition of the doping agent increases the number of Nb and/or Ta sites on the surface of the catalyst and changes the catalyst properties. It is well known that Nb or Ta have a predominant function with regard to selectivity of the oxidation of propane into acrylic acid. When the complete amount of Nb or Ta is introduced during the synthesis, it's difficult to control the Nb or Ta amount present at the surface of the solid. The niobium or tantalum doping allows controlling the amount of Nb or Ta at the surface of the catalyst, improving its catalytic performance. It has been shown that the undoped Mo-V-(Te and/or Sb)-O catalyst optionally containing Nb and/or Ta, presented a selectivity to acrylic acid lower than 40% while selectivities to acrylic acid of about 60% can be obtained on Nb or Ta-doped catalysts.

Thus, in a first aspect, the present invention provides a process for preparing an improved catalyst having the formula (I) :

$$Mo_1V_a(Te \text{ and/or } Sb)_b(Nb \text{ and/or } Ta)_cSi_dO_x \qquad (I)$$

Wherein a is between 0.006 and 1
b is between 0.006 and 1
c is between 0.001 and 0.5
d is between 0 and 3.5

and x is dependent on the oxidation state of the other elements,
said process comprising, in a first step, the synthesis by hydrothermal synthesis method of a Mo-V-(Te and/or Sb)-O mixed metal oxide comprising more than 50% by weight of orthorhombic phase, optionally containing Nb and/or Ta and/or Si, followed in a second step, by the addition to the mixed metal oxide issued from the first step, of a doping agent constituted by at least one component selected from Nb and Ta

In a second aspect, the present invention provides catalysts obtainable by the process according to the first aspect of the invention.

In a third aspect, the present invention provides a process for producing acrylic acid which comprises subjecting propane to a vapour phase catalytic oxidation reaction in the presence of a catalyst produced by the process according to the first aspect of the invention.

The present invention is described in detail below.

Detailed description of the invention

The improved catalyst prepared by the process of the present invention has the empirical formula (I) :

$$Mo_1V_a(Te \text{ and/or } Sb)_b(Nb \text{ and/or } Ta)_cSi_dO_x \qquad (I)$$

Wherein a is between 0.006 and 1
b is between 0.006 and 1
c is between 0.001 and 0.5
d is between 0 and 3.5

and x is dependent on the oxidation state of the other elements.

Preferably, a is between 0.1 and 0.5
b is between 0.01 and 0.3
c is between 0.001 and 0.25
d is between 0 and 1.6

and x is dependent on the oxidation state of the other elements. The amount of Nb and/or Ta, expressed by c, is the sum of the amount optionally present in the mixed metal oxide issued from the first step of the process (c') and the amount added during the second step of the process (c") :

Preferably, c' is between 0 and 0.15
c" is between 0.001 and 0.1

The preferred catalyst prepared by the process of the invention has the empirical formula

$$Mo_1V_aSb_bNb_cSi_dO_x \qquad (II$$

Wherein a, b, c and d may vary in the ranges defined above.

In the first step of the process of the invention, the mainly orthorhombic phase of a Mo-V-(Te and/or Sb)-O mixed metal oxide, optionally also containing Nb and/or Ta and/or Si is provided by hydrothermal synthesis method (HTT) as described for example in Applied Catalysis A : General 194-195 (2000) 479-485.

The mixed metal oxides are generally characterized by their X-ray diffraction patterns. Many authors have described the phases that may be present, for example in: Applied Catalysis A:General 232 (2002) 77-92 ; Applied Catalysis A: General 244 (2003) 359-370 ; Catalysis Letters Vol. 74 N°3-4 (2001) 149-154 ; Catalysis Surveys from Japan Vol. 6, N°1/2 (October 2002) 33-44 ; Chem. Mater. (2003) Vol. 15, N°11, 2112-2114.

The X-ray patterns show mixtures of phases, such as orthorhombic, hexagonal or also $MoO_5$ phases. It is well known that hydrothermal synthesis method leads mainly to orthorhombic phase while dry-up method leads to a mixture of orthorhombic and hexagonal phases. Mainly orthorhombic phase in the present invention means that the mixed metal oxide comprises more than 50% by weight of orthorhombic phase in the crystallized solid, preferably more than 70% and more preferably more than 80%. The amount in orthorhombic phase may be determined after calibration with the pure phases, according to the method of washing and weighting described in the Japanese Laid-Open Patent application Publication N°10-330343 and the X-ray diffraction patterns as described in WO 2004/105938.

A wide range of starting materials including, for example, oxides, nitrates, halides or oxyhalides, alkoxides, acetylacetonates or organometallic compounds may be used.

For example, ammonium molybdate, ammonium paramolybdate or ammonium heptamolybdate may be used for the source of molybdenum in the catalyst. However, compounds such as $MoO_3$, $MoO_2$ $MoCl_5$, $MoOCl_4$, $Mo(OC_2H_5)_5$, molybdenum acetylacetonate, phosphomolybdic acid and silicomolybdic acid may also be utilized.

Similarly, ammonium metavanadate may be utilized for the source of vanadium in the catalyst. However, compounds such as $V_2O_5$, $V_2O_3$, $VOCl_3$, $VCl_4$, $VOSO_4$, $VO(C_2H_5)_3$, vanadium or vanadyl acetylacetonate may also be utilized.

The tellurium source may include telluric acid, $TeCl_4$, $Te(OC_2H_5)_5$, $Te(OCH(CH_3)_2)_4$ and $TeO_2$.

The antimony source may include antimony trioxide, $Sb_2(SO_4)_3$, $SbCl_3$ or $SbCl_5$.

The niobium source may include niobium hydrogen oxalate, ammonium niobium oxalate, $Nb_2O_5$, $NbCl_5$, niobic acid or $Nb(OC_2H_5)_5$, $Nb(O-nBu)_5$, niobium tartrate ...

The tantalum source may include tantalic acid, tantalum oxalate, $TaCl_5$ or $Ta_2O_5$

Optionally, colloidal silica or polysilicic acid may be used.

In the present invention, an aqueous mixture containing Mo, V, Te and/or Sb metal ions in the appropriate atomic ratio is prepared by mixing solution of the possible starting materials described above in water. The temperature is generally of from 20°C to 100°C, preferably from 20°C. to 80°C. Optionally, silica in the form of colloidal silica or polysilicic acid and/or niobium or tantalum source may be added to this solution.

A solution or a slurry is formed. After the solution or the slurry is well stirred, it is introduced for example into a stainless steel autoclave and a reaction is carried out at a temperature in the range of 130°C to 260°C for a duration of 24 to 72 hours. A temperature in the range of 150°C to 200°C is preferred. The reaction provides a black solid, which is washed and dried. Drying methods include, without limitation, vacuum drying, freeze drying, spray drying, rotary evaporation and air drying.

The solid may further be subjected to calcination. The calcination may be conducted in an oxygen-containing atmosphere or in the substantial absence of oxygen, e.g. in an inert atmosphere or in vacuum. Suitable examples of inert atmosphere include without limitation nitrogen, argon, xenon, helium or mixtures thereof. Preferably, the inert atmosphere is nitrogen. The oxygen containing atmosphere or the inert atmosphere may flow over the surface of the catalyst or may not flow thereover.

Preferably, calcinations under static air at a temperature in the range of 250°C to 350°C for at least 10 minutes and under nitrogen at a temperature in the range of 550°C to 700°C for about at least 1 hour are conducted. More particularly, calcination is carried out under static air at 320°C for at least 20 minutes and then under nitrogen at 600° for 2 hours.

The mixed metal oxide synthetized by hydrothermal method comprises mainly orthorhombic phase, but may contain a little amount of hexagonal phase. Further treatments such as for example washing with hydrogen peroxide, oxalic acid, nitric acid solutions may optionally be applied to increase the content in orthorhombic phase.

Hydrothermal method has the advantage to allow the formation of a mainly orthorhombic phase, even in the absence of Nb or Ta in the mixed metal oxide.

Preferably, the mixed metal oxide issued from the first step doesn't contain Nb nor Ta.

The solid issued from the first step may be crushed in order to obtain shorter needles.

The second step of the process of the invention comprises the addition to the metal oxide issued from the first step of a doping agent constituted by at least one component selected from Nb and Ta.

The addition of the doping agent may be carried out by impregnation with solutions containing a source of Nb and/or Ta. The Nb or Ta solutions may be prepared with the sources of Nb or Ta described above, or they may be commercial solutions. An appropriate concentration of these solutions is used in order to obtain the appropriate atomic ratio in the doped solid.

Typically, a mixture of the mixed metal oxide issued from the first step with an impregnation solution is stirred at room temperature during about one hour. The impregnation may be also conducted under a slightly elevated temperature. The mixture is then dried by any suitable method known in the art as described above. Generally, it is dried at a temperature of 70°C to 100°C, preferably at about 80°C during at least 2 hours.

The addition of the doping agent may be carried out alternatively by physical mixed method, e.g. mixing or crushing a Mo-V-(Sb and/or Te)-O sample, optionally containing Nb and/or Ta and/or Si, with a solid Nb or Ta oxide or a mixture of Nb oxide and Ta oxide. The mixing time is typically 5 to 15 minutes. Any other suitable method known in the art to mix solids may be used.

Preferably, the addition of the doping agent is done by impregnation with solutions containing a source of Nb and/or Ta.

The doped mixed metal oxide issued from the second step of the process may be used as a final catalyst, but it may further be subjected to calcination. The calcination may be conducted in an oxygen-containing atmosphere or in the substantial absence of oxygen, e.g. in an inert atmosphere or in vacuum. Suitable examples of inert atmosphere include without limitation nitrogen, argon, xenon, helium or mixtures thereof. Preferably, the inert atmosphere is nitrogen. The oxygen containing atmosphere or the inert atmosphere may flow over the surface of the catalyst or may not flow thereover. The calcination is usually performed at a temperature of from 200°C to 700°C, preferably from 300°C to 600°C. The calcination is performed for from 1 hour to 4 hours, preferably for from 1 hour to 2 hours.

In one mode of operation, the calcination is performed in two stages. In the first stage, the solid is calcined in an oxidizing environment (e.g. air) at a temperature of from 200°C to 400°C, preferably from 250°C to 350°C for from 1 hour to 4 hours. In the second stage, the material issued from the first stage is calcined in an inert atmosphere at a temperature of from 400°C to 700°C , preferably, from 500°C to 600°C for from 1 hour to 2 hours.

Further calcination in static air and/or nitrogen flow provides a solid Nb-and/or Ta-doped Mo-V-(Sb and/or Te)-O catalyst.

One preferred process consists in preparing a Mo-V-Sb-O mixed oxide by hydrothermal method and activation in air and nitrogen, followed by impregnation with a niobium solution and calcination in air and nitrogen.

In a second aspect, the present invention provides catalysts obtainable by the process according to the first aspect of the invention.

The catalyst may be used by itself as a solid catalyst for the production of acrylic acid from propane, but may be formed into a catalyst together with a suitable carrier such as silica, alumina, titania, aluminosilicate, diatomaceous earth or zirconia. Further, it may be molded into a suitable shape and particle size depending upon the scale or system of the reactor.

In a third aspect, the present invention provides a process for producing acrylic acid which comprises subjecting propane to a vapour phase catalytic oxidation reaction in the presence of a catalyst produced by the process according to the first aspect of the invention.

As a starting material gas to be supplied to the reaction system, a gas mixture comprising a steam containing propane and a molecular oxygen containing gas is usually used. However, the steam containing propane and the oxygen containing gas may be alternately supplied to the reaction system.

It is preferred to employ a starting material gas, which contains steam.

Further, as a diluting gas, an inert gas such as nitrogen, argon, or helium may be supplied. The molar ratio propane : oxygen : diluting gas : ($H_2O$) in the starting material gas in generally 1 : 0.05-3 : 1 -10 : 1-10, preferable 1 : 0.05-2 : 1-10 : 1-10 and more preferably 1 : 0.1-1 : 1-5 : 1-5.

To incorporate molecular oxygen into the feed gas, such molecular oxygen may be pure oxygen gas. However, it is usually more economical to use an oxygen containing gas such as air. It is important that propane and oxygen concentrations in the feed gases be maintained at the appropriate levels to minimize or avoid entering a flammable regime within the reaction zone or especially at the outlet of the reactor zone. It is also possible to carry out the vapour phase catalytic reaction in the absence of molecular oxygen. In such a case, it is preferred to adopt a method wherein a part of the catalyst is appropriately sent to an oxidation regenerator to be regenerated, and then returned to the reaction zone for reuse. The regeneration method of the catalyst, such as described in WO 04/0246665 or WO 04/0246666 may be used.

The reaction system may be a fixed bed system or a fluidized bed system. However, a fluidized bed system may preferably be employed whereby it is easy to control the reaction temperature.

The process may be practiced in a single pass mode -only fresh feed is fed to the reactor-, or in a recycle mode -at least a portion of the reactor effluent is returned to the reactor-.

General conditions for the process are as follows: The reaction temperature can vary from 200 to 500°C, but is usually in the range of from 250 to 450°C, more preferably 350 400°C. The reaction can be conducted usually under atmospheric pressure, but may be conducted under a slightly elevated pressure or slightly reduced pressure. Typical pressures are in the range of from 1.01 $10^4$ to 1.01 $10^6$ Pa, preferably from 5.05 $10^4$ to 5.05 $10^5$. The average contact time with the catalyst can be from 0.01 to 90 seconds, preferably from 0.1 to 30 seconds.

When the oxidation reaction of propane is conducted by the process of the present invention, carbon monoxide, carbon dioxide, acetic acid, acetone... may be produced as by-products, in addition to acrylic acid and propylene. The catalyst operates efficiently, significantly avoiding undesirable reactions such as further oxidation and favouring the selective formation of acrylic acid.

The present invention will be explained below in more detail by reference to examples and comparative examples, but the invention should not be construed as being limited to these examples.

In the following examples, the conversion of propane and the selectivity to acrylic acid are represented by the following formulas:

$$\text{Conversion of propane (\%)} = \text{moles of consumed propane/moles of supplied propane} \times 100$$

$$\text{Selectivity to acrylic acid(\%)} = \text{moles of formed acrylic acid/moles of supplied propane} \times 100$$

EXAMPLES

CATALYST PREPARATION

Example 1 : Preparation of Mo-V-Sb-O

The mixed metal oxide with preparative composition of Mo: V: Sb = 6: 2: 1 was prepared by hydrothermal method. First, an amount of 5.35 g of $(NH_4)_6MO_7O_{24}\cdot4H_2O$ (WAKO Chemicals, 99%) was dissolved in 20 ml of water at 80°C. Then, an amount of 1.34 g of $Sb_2(SO_4)_3$ (SOEKAWA Chemicals, 99%) were successively added to the aqueous solution. This suspension was stirred for 15 min. Finally, an aqueous solution of 2.63 g of $VOSO_4\cdot nH_2O$ (MITSUWA Chemicals, assay 62%) in 10 ml of distilled water was added to the dark blue suspension, (not completely dissolved at this stage). After 15 min of stirring, the slurry was introduced in the Teflon inner tube in a stainless steel autoclave. The autoclave was sealed and heated at 175 °C for 24 h. The black solid obtained was washed with distilled water and dried at 80 °C for 12 h. It was first calcined in static air at 320 °C for 20 min and then under nitrogen flow (50 ml/min) at 600 °C for 2 h.

2 batches of catalysts have been prepared. They will be called the MVS-S (S stands for Soekawa) or MVS-C (C stands for CBMM) samples depending on the following treatments. For the following impregnations, the samples were crushed for 5 minutes, in order to obtain shorter needles.

Example 2 : Preparation of Nb-doped Mo-V-Sb-O by niobium hydrogen oxalate (SOEKAWA Chem.)

Nb-doped Mo-V-Sb-O catalysts (with preparative Nb/Mo atomic ratios 0.008/6, 0.016/6, and 0.032/6) was prepared by impregnation (Volume of solution: 10 ml) of the MVS-S sample (1g) with colloidal solutions of Nb $(HC_2O_4)_5 \cdot n H_2O$ (SOEKAWA Chem., Assay $Nb_2O_5$ 14.9%). The sample was stirred for 1 h at room temperature and dried at 80°C for 12h. It was first calcined in static air at 320 °C for 20 min and then under nitrogen flow (50 ml/min) at 600 °C for 2 h. After activation samples will be called the Nb-S-0.008, Nb-S-0.016 and Nb-S-0.032 sample, respectively.

Example 3 : Preparation of Nb-doped Mo-V-Sb-O by ammonium niobium oxalate (CBMM -Lot Number AD/3084)

Nb-doped Mo-V-Sb-O catalysts (with preparative Nb/Mo atomic ratios 0.032/6 and 0.064/6) were prepared by impregnation of the MVS-C catalyst sample (1 g) with solutions (Volume 10ml) of $NH_4 [NbO(C_2O_4)_2(H_2O)_2] \cdot n H_2O$ (CBMM, Assay Nb 17.8%). The samples was(were ?) stirred for 1h at room temperature and dried at 80°C for 12h. It was first calcined in static air at 320 °C for 20 min and then under nitrogen flow (50 ml/min) at 600°C for 2 h. After activation samples will be called the Nb-C-0.032 and Nb-C-0.064 sample, respectively.

Example 4: Preparation of Nb-doped Mo-V-Sb-O by physical mixed method

An amount of 0.1g $Nb_2O_5$ powder (from WAKO) added to MVS-C sample (0.5g) and mixed for 5 min (while crushing in an agate mortar). It was calcined under nitrogen flow (50 ml/min) at 600 °C for 2 h. Catalyst weight was decreased from 0.6 to 0.56g. It will be .called MVS-C-$Nb_2O_5$ sample.

Example 5 (comparative)

For comparative purpose, MVS-S catalyst sample was treated with water only and with a solution of 0.04M $(NH_4)_2C_2O_4$ (KATAYAMA Chemicals, 99.5%) in the same way as that described in example 2. These will be called MVS-$H_2O$ and MVS-AO (AO=Ammonium Oxalate), respectively.

CATALYSTS CHARACTERIZATION AND ACTIVITY TEST

Powder XRD patterns were recorded with a Rigaku Ris-lvb diffractometer using Cu Ka radiation. Samples were ground and put on a horizontal sample holder. XRD patterns were recorded in the 2 to 60 ° range.

Propane oxidation was carried out at atmospheric pressure in a conventional flow system with a fixed-bed Pyrex tubular reactor in the temperature range of 300 to 380 °C. The feed composition was $C_3H_8$: $O_2$: $N_2$: $H_2O$ = 6.5: 10: 38.5: 45 vol. %. The amount of catalyst was 500 mg. The total flow rate was 20ml/min. Both reactants and products were analyzed by an on-line GC system equipped with the following columns: (1) Gaskuropack 54 to separate hydrocarbons and $CO_2$, (2) Molecular Sieve 13X to separate $O_2$, $N_2$ and CO and (3) Porapak QS to separate oxygenated products (acetone, acetic acid and acrylic acid). Blank runs showed that under the experimental conditions used in the present study, homogeneous gas-phase reaction was negligible.

EVALUATION AND RESULTS

Example 6 :

Catalysts of example 1 (MVS-S and MVS-C sample), example 2 (Nb-S-0.008, Nb-S-0.016 and Nb-S-0.032 sample) and example 3 (Nb-C-0.032 and Nb-C-0.064 sample) were evaluated in propane selective oxidation to acrylic acid, as described above. The results are shown in table 1.

Table 1

| Catalyst | Reaction Temperature, °C | Conversion, % | | Selectivity, % | | | | | |
|---|---|---|---|---|---|---|---|---|---|
| | R.T. (°C) | $C_3H_8$ | $O_2$ | AA | PN | Ace | AcA | CO | $CO_2$ |
| Example 1 MVS-S (comparative) | 300 | 15.8 | 26.6 | 35 | 13 | 5 | 18 | 15 | 14 |
| | 320 | 28.6 | 50.5 | 34 | 8 | 2 | 21 | 18 | 17 |
| | 340 | 41.7 | 82.5 | 27 | 6 | 1 | 22 | 22 | 22 |
| Example 2 Nb-S-0.008 | 300 | 18..2 | 27..1 | 45 | 13 | 5 | 15 | 11 | 11 1 |
| | 320 | 26..5 | 44.5 | 42 | 9 | 2 | 17 | 15 | 14 |
| | 340 | 39..0 | 73.9 | 33 | 6 | 1 | 19 | 21 | 20 |
| Example 2 Nb-S-0.016 | 280 | 8..5 | 11..7 | 42 | 22 | 12 | 11 | 7 | 7 |
| | 300 | 15..6 | 22..8 | 48 | 16 | 6 | 12 | 10 | 8 |
| | 320 | 24..5 | 37..3 | 49 | 11 | 3 | 14 | 12 | 10 |
| | 340 | 35.6 | 60.5 | 46 | 8 | 1 | 15 | 15 | 14 |
| | 350 | 40.4 | 74.4 | 42 | 7 | 1 | 16 | 18 | 16 |
| | 360 | 45.7 | 87.8 | 37 | 8 | 1 | 16 | 20 | 18 |
| Example 2 Nb-S-0.032 | 280 | 5.4 | 7.2 | 45 | 28 | 12 | 6 | 4 | 5 |
| | 300 | 9.8 | 13.5 | 51 | 23 | 7 | 8 | 5 | 6 |
| | 320 | 14.8 | 22.1 | 57 | 17 | 4 | 9 | 7 | 6 |
| | 340 | 22.0 | 33.6 | 59 | 14 | 2 | 9 | 9 | 7 |
| | 360 | 29.0 | 47.3 | 58 | 11 | 1 | 10 | 10 | 9 |
| | 370 | 35.9 | 59.0 | 56 | 9 | 1 | 11 | 12 | 11 |
| | 380 | 42.3 | 74.4 | 52 | 7 | 1 | 11 | 16 | 14 |
| Example 1 MVS-C (comparative) | 280 | 8.2 | 12.8 | 36 | 15 | 15 | 16 | 8 | 9 |
| | 300 | 16.3 | 28.8 | 36 | 11 | 4 | 20 | 13 | 15 |
| | 320 | 23.9 | 43.6 | 35 | 9 | 3 | 21 | 15 | 17 |
| | 340 | 34.9 | 67.0 | 30 | 7 | 1 | 22 | 19 | 21 |
| Example 3 Nb-C-0.032 | 280 | 9.3 | 15.1 | 40 | 16 | 7 | 15 | 10 | 11 |
| | 300 | 14.9 | 25.3 | 43 | 13 | 4 | 16 | 11 | 13 |
| | 320 | 23.2 | 41.5 | 42 | 10 | 2 | 17 | 15 | 15 |
| | 340 | 33.8 | 63.8 | 38 | 7 | 1 | 18 | 17 | 18 |
| | 360 | 44.1 | 87.3 | 31 | 6 | 1 | 18 | 22 | 23 |
| Example 3 Nb-C-0.064 | 280 | 7.6 | 11.9 | 41 | 20 | 6 | 13 | 9 | 11 |
| | 300 | 14.1 | 24.8 | 45 | 15 | 3 | 15 | 10 | 13 |
| | 320 | 22.1 | 39.7 | 41 | 11 | 1 | 16 | 14 | 16 |
| | 340 | 31.2 | 58.6 | 37 | 9 | 1 | 16 | 18 | 19 |
| | 360 | 40.1 | 80.6 | 30 | 7 | 0 | 16 | 23 | 24 |

AA=acrylic acid, PEN=propylene, Ace=acetone, AcA=acetic acid

The results show that the impregnation treatment leads to an increase of selectivity in Acrylic acid but also for propylene. Over all selectivity in Acrylic Acid + Propylene (both valuable products) is increased with only a slight loss of conversion at the highest niobium loading. The activation energy for the conversion of propane is not affected by the impregnation treatment, indicating that niobium in this case does not interfere with propane activation. The improvement of selectivity in doped catalyst is then related to the reduced combustion or degradation of acrylic acid.

Example 7 :

The mechanical mixture of catalyst and niobium oxide of example 4 (MVS-C-$Nb_2O_5$ sample) was compared with a mechanical mixture of catalyst MVS-C and silicon carbide (well known to be inactive in the reaction). An increase of selectivity can be also observed in propane oxidation over MVS-C-$Nb_2O_5$, as it is shown in table 2.

Table 2

| Catalyst | Reaction Temperature, °C | Conversion, % | | Selectivity, % | | | | | |
|---|---|---|---|---|---|---|---|---|---|
| | R.T. (°C) | $C_3H_8$ | $O_2$ | AA | PEN | Ace | AcA | CO | $CO_2$ |
| Example 4 MVS-C-$Nb_2O_5$ | 280 | 6.4 | 12.3 | 39 | 21 | 6 | 13 | 9 | 12 |
| | 300 | 16.2 | 23.0 | 43 | 16 | 3 | 14 | 11 | 13 |
| | 320 | 22.1 | 35.4 | 44 | 12 | 2 | 16 | 11 | 15 |
| | 330 | 23.6 | 43.6 | 42 | 11 | 1 | 16 | 13 | 17 |
| | 350 | 35.6 | 66.4 | 38 | 8 | 1 | 17 | 17 | 20 |
| | | | | | | | | | |
| Comparative MVS-C-SiC- | 280 | 9.5 | 14.6 | 35 | 14 | 10 | 17 | 12 | 12 |
| | 300 | 16.8 | 26.3 | 37 | 11 | 6 | 20 | 12 | 14 |
| | 320 | 22.2 | 37.2 | 37 | 10 | 4 | 21 | 14 | 15 |
| | 340 | 31.4 | 57.8 | 32 | 8 | 2 | 22 | 18 | 19 |
| | 360 | 40.9 | 78.7 | 27 | 6 | 1 | 22 | 22 | 22 |

Example 8 :

In this example, catalysts of comparative example 5 (MVS-$H_2O$ and MVS-AO sample) were compared to catalyst Nb-S-0.016 of example 2 in propane selective oxidation to acrylic acid, as described above. The results are shown in table 3.

Table 3

| Catalyst | Reaction Temperature, °C | Conversion, % | | Selectivity, % | | | | | |
|---|---|---|---|---|---|---|---|---|---|
| | R.T. (°C) | $C_3H_8$ | $O_2$ | AA | PEN | Ace | AcA | CO | $CO_2$ |
| Example 5 MVS-H2O (comparative) | 280 | 10.9 | 18.7 | 36 | 14 | 7 | 17 | 12 | 14 |
| | 300 | 17.6 | 29.7 | 35 | 11 | 4 | 20 | 13 | 16 |
| | 320 | 25.2 | 45.8 | 33 | 9 | 2 | 21 | 16 | 19 |
| | 340 | 34.8 | 65.0 | 29 | 7 | 1 | 22 | 19 | 21 |
| | 360 | 42.6 | 83.9 | 24 | 6 | 1 | 22 | 22 | 25 |
| | | | | | | | | | |
| Example 5 MVS-AO (comparative) | 280 | 10.1 | 18.1 | 36 | 14 | 7 | 17 | 11 | 15 |
| | 300 | 16.3 | 31.4 | 35 | 10 | 4 | 19 | 15 | 17 |
| | 320 | 28.3 | 51.6 | 32 | 8 | 2 | 21 | 17 | 21 |
| | 340 | 35.5 | 72.3 | 27 | 6 | 1 | 22 | 19 | 24 |
| | 350 | 41.4 | 83.2 | 24 | 6 | 1 | 22 | 21 | 26 |
| | | | | | | | | | |

(continued)

| Catalyst | Reaction Temperature, °C | Conversion, % | | Selectivity, % | | | | | |
|---|---|---|---|---|---|---|---|---|---|
| | R.T. (°C) | $C_3H_8$ | $O_2$ | AA | PEN | Ace | AcA | CO | $CO_2$ |
| Example 2 Nb-S-0.016 | 280 | 8.5 | 11.7 | 42 | 22 | 12 | 11 | 7 | 7 |
| | 300 | 15.6 | 22.8 | 48 | 16 | 6 | 12 | 10 | 8 |
| | 320 | 24.5 | 37.3 | 49 | 11 | 3 | 14 | 12 | 10 |
| | 340 | 35.6 | 60.5 | 46 | 8 | 1 | 15 | 15 | 14 |
| | 350 | 40.4 | 74.4 | 42 | 7 | 1 | 16 | 18 | 16 |
| | 360 | 45.7 | 87.8 | 37 | 8 | 1 | 16 | 20 | 18 |

No improvement in selectivity to acrylic acid is recorded in the experiment with only water and with ammonium oxalate solution.

REFERENCE EXAMPLES

Reference example 1 :

Reference catalysts, having the same overall chemical compositions but having all the niobium added during their synthesis, have been prepared using the following hydrothermal procedure.
The target catalyst compositions are the following:

$Mo_6V_2Sb_1O_x$
$Mo_6V_2Sb_1Nb_{0.008}O_x$,
$Mo_6V_2Sb_1Nb_{0.016}O_x$
$Mo_6V_2Sb_1Nb_{0.032}O_x$
$Mo_6V_2Sb_1Nb_{0.064}O_x$

*Hydrothermal procedure*

5.35 g ($30.10^{-3}$ mol of Mo) of $(NH_4)_6Mo_7O_{24}.nH_2O$ (Wako, research grade) is dissolved in 20 ml of distilled water in a beaker (50ml size) with stirring using a magnetic stirrer. This solution is then heated up to 80 °C using a hot-plate magnetic stirrer. To the solution that Mo compound is completely dissolved at the temperature of 80 °C, 1.34 g ($5.10^{-3}$ mol of Sb) of $Sb_2(SO_4)_3$ (Soekawa, research grade, anhydride) powder (without grinding) is added directly at once. The resulting slurry is stirred for 15 minutes with keeping the temperature of 80 °C. The slurry colour becomes dark green after the 15 minutes stirring. Separately, an aqueous vanadium solution is prepared by dissolving 2.64 g ($10.10^{-3}$ mol of V) of $VOSO_4.nH_2O$ (Mitsuwa Chemicals, research grade, assay 62.0%) in 10 ml of distilled water in a beaker (50ml size) with hand-stirring at room temperature. The vanadium solution is added at once to the Mo-Sb slurry with vigorous stirring and the mixed solution is stirred for 15 minutes at 80 °C.
Separately, an aqueous Nb solution is prepared by dissolving the desired amount of $Nb(HC_2O_4)_5$ $nH_2O$ (Soekawa, research grade, assay $Nb_2O_5$ 14.92%) in 10 ml of distilled water in a beaker (50ml size) with hand-stirring at 80 °C: The Nb solution is added at once to the Mo-Sb-V slurry with vigorous stirring and the mixed solution is stirred for 5 minutes at 80 °C. Finally the slurry is introduced into a 70 ml Teflon inner tube of a stainless steel autoclave and heated at 175 °C for 24 hours.
After 24 hours, the autoclave is cooled in a water flow for about 60 minutes. The dark blue powder obtained is separated from the solution by filtration (filtration paper #4A), and then washed with about 500 ml of distilled water. Finally the black material is dried in an oven at 80 °C for 12 hours.
The dried solid is gently ground for about 1 minute using an agate mortar. Then the powder is pre-calcined in an alumina crucible in static air at 320 °C for 20 minutes using a muffle furnace. After the calcination the sample is cooled in the furnace by stopping the heating. After that, the sample (2g, dried) is loaded in a quartz tube reactor and calcined in nitrogen flow (50 ml/min) at 600 °C for 2 hours in a tubular furnace. The heating rate is 10 °C/min from room temp. to 600 °C and then cooled without control.
Before catalytic test, the catalysts are ground strongly for 5 minutes again using an agate mortar.

*Evaluation and results*

All the catalysts were tested in selective propane oxidation reaction in the same conditions as described above.

The table 4 presents the catalytic results (The values given in this table are obtained doing an average of the selectivity for each species for the experiments for which the carbon balance was between 95 and 105%.).

Table 4

| catalyst | T (°C) | Conv, % | Yield, % | | Selectivity, % | | | | | |
|---|---|---|---|---|---|---|---|---|---|---|
| | | | PEN | AA | AA | PEN | Ace | AcA | $CO_2$ | CO |
| MoVSb | 300 | 12.0 | 3.7 | 31.3 | 15.1 | 0.7 | 20.5 | 12.4 | 14.5 |
| | 320 | 17.0 | 5.2 | 30.4 | 12.8 | 0.6 | 22.1 | 14.1 | 16.8 |
| | 340 | 20.7 | 6.0 | 28.8 | 11.8 | 0.5 | 22.1 | 16.7 | 18.4 |
| | 360 | 28.0 | 5.4 | 25.3 | 10.7 | 0.4 | 23.6 | 21.7 | 23.8 |
| | 380 | 28.8 | 5.2 | 18.1 | 10.0 | 0.2 | 21.7 | 22.9 | 26.5 |
| $MoVSbNb_{0.008}$ | 300 | 9.6 | 2.6 | 27.0 | 13.7 | 5.7 | 23.1 | 12.6 | 17.9 |
| | 320 | 14.7 | 3.7 | 24.9 | 12.2 | 3.0 | 26.4 | 15.6 | 17.8 |
| | 340 | 22.9 | 5.1 | 22.4 | 10.2 | 1.6 | 25.2 | 19.1 | 21.5 |
| | 360 | 33.8 | 4.4 | 13.2 | 8.5 | 0.7 | 17.6 | 28.5 | 31.6 |
| | 380 | 20.9 | 1.9 | 8.9 | 8.8 | 0.5 | 22.4 | 26.1 | 33.4 |
| $MoVSbNb_{0.016}$ | 300 | 17.4 | 5.4 | 22.2 | 9.5 | 5.7 | 28.1 | 16.5 | 18.0 |
| | 320 | 19.3 | 3.2 | 16.8 | 6.6 | 2.1 | 35.3 | 18.3 | 21.4 |
| | 340 | 27.3 | 4.3 | 15.6 | 6.5 | 1.6 | 31.3 | 21.0 | 24.1 |
| | 360 | 35.6 | 3.4 | 9.6 | 5.4 | 0.9 | 28.9 | 27.3 | 30.3 |
| | 380 | 42.7 | 2.7 | 6.4 | 5.7 | 0.9 | 22.1 | 31.8 | 33.1 |
| $MoVSbNb_{0.032}$ | 300 | 11.2 | 3.3 | 29.4 | 15.2 | 7.5 | 20.0 | 11.4 | 16.5 |
| | 320 | 16.3 | 5.0 | 30.5 | 13.1 | 3.8 | 23.3 | 14.1 | 15.1 |
| | 340 | 22.3 | 5.9 | 26.3 | 10.6 | 2.1 | 23.7 | 17.6 | 19.7 |
| | 360 | 27.4 | 5.6 | 20.2 | 9.6 | 1.2 | 24.6 | 20.8 | 23.6 |
| | 380 | 28.4 | 2.5 | 8.3 | 8.9 | 0.5 | 22.2 | 27.0 | 33.0 |
| $MoVSbNb_{0.064}$ | 300 | 10.6 | 2.8 | 26.2 | 10.5 | 7.9 | 27.0 | 12.8 | 15.5 |
| | 320 | 22.1 | 5.3 | 24.1 | 9.0 | 3.8 | 28.5 | 16.2 | 18.5 |
| | 340 | 25.5 | 4.3 | 16.8 | 6.9 | 1.9 | 28.1 | 22.6 | 23.7 |
| | 360 | 33.4 | 2.6 | 7.8 | 5.5 | 0.8 | 27.7 | 27.2 | 31.1 |
| | 380 | 36.7 | 1.5 | 4.1 | 5.2 | 0.7 | 21.4 | 31.9 | 36.8 |

The presence of niobium in the composition of the mixed metal oxide does not improve the acrylic acid selectivity when niobium is introduced directly during the hydrothermal synthesis.

Reference example 2 :

Reference catalysts have been prepared using the dry-up method followed by impregnation with solutions containing a source of Nb or Ta.

*Dry-up procedure* - *Expected composition: $Mo_1V_{0.3}Sb_{0.15}Nb_{0.1}Si_{0.93}O_x$*

Slurry preparation

In a Rayneri Trimix are introduced the following products : 295 g of niobic acid (HY-340 CBMM, 80% $Nb_2O_5$), 660 g of oxalic acid di-hydrated (Prolabo), 5 litres of demineralised water. The dissolution of niobic acid requires two hours at 65°C. The solution is then cooled and kept. All the following operations are achieved under nitrogen atmosphere to control the oxidation state of elements. In a Rayneri Trimix are introduced 3090 g of ammonium heptamolybdate (Starck), 615 g of ammonium metavanadate (GFE), 385 g of antimony oxide ($Sb_2O_3$, Campine), 9750 g of demineralised water. The solution is heated under stirring at 97-100°C during three hours after stabilisation of temperature. The resulting mixture is dark blue opaque. Then 355 g of hydrogen peroxide 30wt% are added. The solution colour turns to limpid orange. 2455 g colloidal silica Ludox (Grace, AS-40, 40wt%) are added without any modification of aspect of the solution. Finally, the previous solution of oxalic acid and niobic acid is added. The mixture becomes turbid and the colour turns to yellow-green. The measured dried material ratio (by infra-red dessiccator) is 29 wt%.

The solution remains under stirring, still at the same temperature during 30 minutes. Heating is then stopped. Immediately after, the mixture is spray-dried.

Spray-drying

For spray-drying, a home-modified NIRO is used. Drying flowing gas is nitrogen. The nozzle is ultrasonic type (ultrasonic frequency: 20 kHz). The feeding tank is continuously stirred and heated at 60°C with a thermostated bath. Operation conditions are the following : Inlet gas temperature 210-215 °C - Outlet gas temperature 110°C - Feeding flow 5 kg/h - Nitrogen flow 80 m³/h. The evaporation capacity is 3kg/h of water. The resulting green precursor is then dried one night in an oven at 80°C. The precursor is then sieved and the considered valuable fraction is 50-160 $\mu$m.

Thermal treatment

The thermal treatment is achieved with rotating furnace (flask dimensions : 200 mm diameter, 270 mm cylindrical length, 2.5 L effective volume). One extremity is closed. The rotating speed is around 15 r.p.m.

First, for pre-calcination, 3500 g of powder precursor are heated at 310°C with 400 1/h of flowing air during 4 hours. The temperature rate in the solid is 3.5°C/min. Then, for calcination, the solid is heated at 600° under nitrogen (400 l/h) during two hours. The temperature ratio is 3.5°C/min. Nitrogen must be very pure to avoid over-oxidation. After calcination, the solid presents a crystalline structure (both hexagonal and orthorhombic phases).

Catalyst washing

The calcined catalyst is washed with hydrogen peroxide. 500 g of solid are washed during 3 hours at 60°C by a hydrogen peroxide solution (900 g of $H_2O_2$ 30wt% with 8320 g of water). 442g of remaining solid are filtered and washed with demineralised water. It is finally dried in oven at 80°C. During this step, the hexagonal phase is removed.

*Impregration procedure*

Reference Ta and Nb doped catalysts are prepared by impregnation of 20 g of the solid obtained above with solutions of tantalum oxalate (Starck) or niobium oxalate (Starck). The solution volume to be added is calculated from porosity of the solid. The solid is impregnated by dripping the solution on a sample placed on a vibrating table. Then, the doped catalyst is dried at 80°C for 12 h.

*Evaluation and results*

Propane oxidation was carried out at atmospheric pressure in a conventional flow system with a fixed-bed Pyrex tubular reactor at the temperature 380 °C. The feed composition was $C_3H_8$: $O_2$: He-Kr: $H_2O$ = 9: 9: 41: 41 vol. %. The amount of catalyst was 1 g of dried catalyst or 5 g of calcined at 600°C catalyst. The total flow rate was 170ml/min. Both reactants and products were analyzed by micro GC system CP2002 equipped with the following columns (1) Silicaplot to separate hydrocarbons and $CO_2$, (2) Molecula Sieve to separate $O_2$, Kr and CO, and by GC system HP6890 equipped with the following column (3) EC 1000 to separate oxygenated products (acetone, acetic acid and acrylic acid). For each test, carbon balance was drawn up.

The table 5 presents the results concerning the activity test of samples of 1 g of reference Ta or Nb doped catalysts, compared to undoped catalyst.

Table 5

| Sample | Undoped catalyst | Doped catalysts | | | |
|---|---|---|---|---|---|
| | without impregnation | Ta doped Ta 0.02 | Ta doped Ta 0.05 | Nb doped Nb 0.02 | Nb doped Nb 0.05 |
| Conversion $C_3H_8$, % | 24.6 | 26.1 | 25.9 | 21.7 | 12.1 |
| Conversion $O_2$, % | 18.1 | 18.5 | 18.0 | 13.6 | 7.05 |
| Selectivity, % | | | | | |
| AA | 59.8 | 56.2 | 55.5 | 53.6 | 47.3 |
| PEN | 14.2 | 14.7 | 14.5 | 16.8 | 25.9 |
| Ace | 1.22 | 2.05 | 2.12 | 1.11 | 0.82 |
| AcA | 9.15 | 10.2 | 10.9 | 7.76 | 7.05 |
| CO | 8.59 | 8.60 | 8.79 | 11.1 | 10.1 |
| $CO_2$ | 6.63 | 7.93 | 7.8 | 9.13 | 8.16 |

The results show that the impregnation treatment does not lead to an increase of selectivity in acrylic acid. The activation energy for the conversion of propane is not affected by the impregnation treatment regarding Ta-doped catalysts, but it is affected regarding Nb-doped catalysts, indicating that niobium interferes with propane activation.
The table 6 presents the results concerning the activity test of samples of 5 g of the reference Ta or Nb doped catalysts after calcination at 600°C.

Table 6

| Sample | Undoped and calcined catalyst | Doped and calcined catalysts | | | |
|---|---|---|---|---|---|
| | without impregnation | Ta doped Ta 0.02 | Ta doped Ta 0.05 | Nb doped Nb 0.02 | Nb doped Nb 0.05 |
| Conversion $C_3H_8$, % | 31.9 | 21.5 | 11.7 | 24.7 | 13.8 |
| Conversion $O_2$, % | 22.2 | 14.1 | 6.3 | 17.3 | 8.00 |
| Selectivity, % | | | | | |
| AA | 51.8 | 50.1 | 42.7 | 52.1 | 46.6 |
| PEN | 10.6 | 17.2 | 33.1 | 13.5 | 27.8 |
| Ace | 1.66 | 1.90 | 1.21 | 0.99 | 0.95 |
| AcA | 12.9 | 10.3 | 8.0 | 12.3 | 8.5 |
| CO | 12.6 | 11.2 | 7.76 | 11.3 | 8.7 |
| $CO_2$ | 10.1 | 8.80 | 6.6 | 9.40 | 6.78 |

No appreciable improvement in selectivity to acrylic acid is recorded in the experiment with the doped catalysts after calcination.

**Claims**

1. A process for preparing an improved catalyst having the formula (I) :

$$Mo_1V_a(\text{Te and/or Sb})_b(\text{Nb and/or Ta})_cSi_dO_x$$

Wherein a is between 0.006 and 1
b is between 0.006 and 1
c is between 0.001 and 0.5
d is between 0 and 3.5

and x is dependent on the oxidation state of the other elements,
said process comprising, in a first step, the synthesis by hydrothermal synthesis method of a Mo-V-(Te and/or Sub)-O mixed metal oxide comprising more than 50% by weight of orthorhombic phase, optionally containing Nb and/or Ta and/or Si, followed in a second step, by the addition to the mixed metal oxide issued from the first step, of a doping agent constituted by at least one component selected from Nb and Ta

2. A process according to claim 1, wherein

a is between 0.1 and 0.5
b is between 0.01 and 0.3
c is between 0.001 and 0.25
d is between 0 and 1.6

3. A process according to claim 1 or 2, wherein c is the sum of c' and c'', c' being the amount of Nb and/or Ta optionally present in the mixed metal oxide and c'' the amount added during the second step, and

c' is between 0 and 0.15
c'' is between 0.001 and 0.1

4. A process according to any claim 1 to 3, wherein the catalyst has the formula

$$Mo_1V_aSb_bNb_cSi_dO_x \qquad (II)$$

with a, b, c and d defined above.

5. A process according to any claim 1 to 4, wherein the mixed metal oxide issued from the first step is calcined.

6. A process according to any claim 1 to 5, wherein the mixed metal oxide issued from the first step is submitted to further treatments to increase the content in orthorhombic phase.

7. A process according to any claim 1 to 6, wherein the addition of the second step is carried out by impregnation with solutions containing a source of Nb and/or Ta.

8. A process according to any claim 1 to 6, wherein the addition of the second step is carried out by physical mixed method.

9. A process according to any claim 1 to 8, wherein the product issued from the second step is calcined.

10. Catalysts obtainable by the process according to any claim 1 to 9.

11. A process for producing acrylic acid which comprises subjecting propane to a vapor phase catalytic oxidation reaction in the presence of a catalyst produced by the process according to any claim 1 to 9.

**Patentansprüche**

1. Prozess zur Herstellung eines verbesserten Katalysators mit der Formel (I):

$$Mo_1V_a(Te \text{ und/oder } Sb)_b (Nb \text{ und/oder } Ta)_cSi_dO_x$$

wobei

a zwischen 0,006 und 1 liegt
b zwischen 0,006 und 1 liegt
c zwischen 0,001 und 0,5 liegt
d zwischen 0 und 3,5 liegt
und x vom Oxidationszustand der anderen Elemente abhängt,

wobei der Prozess in einem ersten Schritt die Synthese eines Mo-V-(Te und/oder Sb)-O-Mischmetalloxids durch ein hydrothermisches Syntheseverfahren aufweist, wobei das Mo-V-(Te und/oder Sb)-0-Mischmetalloxid mehr als 50 Gewichtsprozent an orthorhombischer Phase aufweist und optional Nb und/oder Ta und/oder Si enthält, gefolgt von einem zweiten Schritt, bei dem zu dem Mischmetalloxid, das aus dem ersten Schritt erhalten wurde, ein Dotierungsstoff hinzugegeben wird, der durch mindestens eine Komponente gebildet wird, die unter Nb und Ta ausgewählt ist.

2. Prozess nach Anspruch 1, wobei

a zwischen 0,1 und 0,5 liegt
b zwischen 0,01 und 0,3 liegt
c zwischen 0,001 und 0,25 liegt
d zwischen 0 und 1,6 liegt.

3. Prozess nach Anspruch 1 oder 2, wobei c die Summe von c' und c" ist, wobei c' die Menge an Nb und/oder Ta ist, die optional in dem Mischmetalloxid vorliegt, und c" die Menge ist, die während des zweiten Schrittes hinzugefügt wird, und

c' zwischen 0 und 0,15 liegt
c" zwischen 0,001 und 0,1 liegt.

4. Prozess nach einem der Ansprüche 1 bis 3, wobei der Katalysator folgende Formel hat:

$$Mo_1V_aSb_bNb_cSi_dO_x \qquad (II)$$

wobei a, b, c und d wie oben definiert sind.

5. Prozess nach einem der Ansprüche 1 bis 4, wobei das Mischmetalloxid, das aus dem ersten Schritt erhalten wird, kalziniert wird.

6. Prozess nach einem der Ansprüche 1 bis 5, wobei das Mischmetalloxid, das aus dem ersten Schritt erhalten wird, weiteren Behandlungen unterzogen wird, um den Gehalt an orthorhombischer Phase zu erhöhen.

7. Prozess nach einem der Ansprüche 1 bis 6, wobei die Hinzufügung des zweiten Schrittes durch Imprägnieren mit Lösungen ausgeführt wird, die eine Quelle von Nb und/oder Ta enthalten.

8. Prozess nach einem der Ansprüche 1 bis 6, wobei die Hinzufügung des zweiten Schrittes durch ein Verfahren des physischen Vermischens ausgeführt wird.

9. Prozess nach einem der Ansprüche 1 bis 8, wobei das Produkt, das aus dem zweiten Schritt erhalten wird, kalziniert wird.

10. Katalysatoren, die durch den Prozess nach einem der Ansprüche 1 bis 9 hergestellt werden können.

11. Prozess zum Herstellen von Acrylsäure, der beinhaltet, Propan einer katalytischen Dampfphasen-Oxidationsreaktion in Gegenwart eines Katalysators zu unterziehen, der durch den Prozess nach einem der Ansprüche 1 bis 9 hergestellt wurde.

**Revendications**

1.  Procédé de préparation d'un catalyseur amélioré de la formule générale (I) :

    $$Mo_1V_a(Te \ et/ou \ Sb)_b(Nb \ et/ou \ Ta)_cSi_dO_x$$

    Dans laquelle a est compris entre 0,006 et 1
    b est compris entre 0,006 et 1
    c est compris entre 0,001 et 0,5
    d est compris entre 0 et 3,5
    et x dépend de l'état d'oxydation des autres éléments,

    ledit procédé comprenant, dans une première étape, la synthèse par un procédé de synthèse hydrothermique d'un oxyde métallique mélangé Mo-V-(Te et/ou Sb)-O qui comprend plus de 50 % en poids d'une phase orthorhombique, qui comprend éventuellement du Nb et/ou du Ta et/ou du Si, suivie, dans une seconde étape, par l'ajout à l'oxyde métallique mélangé produit lors de la première étape d'un agent dopant constitué par au moins un composant choisi par le Nb et le Ta.

2.  Procédé selon la revendication 1, dans lequel :

    a est compris entre 0,1 et 0,5
    b est compris entre 0,01 et 0,3
    c est compris entre 0,001 et 0,25
    d est compris entre 0 et 1,6

3.  Procédé selon la revendication 1 ou 2, dans lequel c représente la somme de c' et de c'', c' étant la quantité de Nb et/ou de TA éventuellement présente dans l'oxyde métallique mélangé et c'' étant la quantité ajoutée lors de la seconde étape, et

    c' est compris entre 0 et 0,15
    c'' est compris entre 0,001 et 0,1

4.  Procédé selon les revendications 1 à 3, dans lequel le catalyseur a la formule

    $$Mo_1V_aSb_bNb_cSi_dO_x \qquad (II)$$

    avec a, b, c et d tels que définis ci-dessus.

5.  Procédé selon l'une quelconque des revendications 1 à 4, dans lequel l'oxyde métallique mélangé obtenu lors de la première étape est calciné.

6.  Procédé selon l'une quelconque des revendications 1 à 5, dans lequel l'oxyde métallique mélangé obtenu lors de la première étape est soumis à d'autres traitements pour augmenter la teneur de la phase orthorhombique.

7.  Procédé selon l'une quelconque des revendications 1 à 6, dans lequel l'ajout de la seconde étape est effectué par une imprégnation avec des solutions qui contiennent une source de Nb et/ou de Ta.

8.  Procédé selon l'une quelconque des revendications 1 à 6, dans lequel l'ajout de la seconde étape est effectué par un procédé physique mixte.

9.  Procédé selon l'une quelconque des revendications 1 à 8, dans lequel le produit obtenu lors de la seconde étape est calciné.

10. Catalyseurs que l'on obtient par le procédé selon l'une quelconque des revendications 1 à 9.

11. Procédé de fabrication d'un acide acrylique, lequel consiste à soumettre du propane à une réaction d'oxydation catalytique en phase vapeur en présence d'un catalyseur produit par le procédé selon l'une quelconque des revendications 1 à 9.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 608838 A
- JP 10330343 A
- US 6060422 A
- US 20030013904 A
- US 20020183198 A
- JP 10028862 A
- WO 2004105938 A
- WO 040246665 A
- WO 040246666 A

### Non-patent literature cited in the description

- *Applied Catalysis A:General,* 2002, vol. 232, 77-92
- *Applied Catalysis A: General,* 2003, vol. 244, 359-370
- *Catalysis Letters,* 2001, vol. 74 (3-4), 149-154
- *Catalysis Surveys from Japan,* October 2002, vol. 6 (1/2), 33-44
- *Chem. Mater.,* 2003, vol. 15 (11), 2112-2114